(19) **Europäisches Patentamt European Patent Office Office européen des brevets**

(11) **EP 2 196 208 A1**

(12) **EUROPEAN PATENT APPLICATION**
published in accordance with Art. 153(4) EPC

(43) Date of publication:
**16.06.2010 Bulletin 2010/24**

(21) Application number: **08834664.8**

(22) Date of filing: **26.09.2008**

(51) Int Cl.:
*A61K 36/18* (2006.01)　　*A61K 8/368* (2006.01)
*A61K 8/97* (2006.01)　　*A61K 31/191* (2006.01)
*A61P 39/06* (2006.01)　　*A61Q 11/00* (2006.01)
*A61Q 13/00* (2006.01)　　*A61Q 15/00* (2006.01)
*A61Q 17/00* (2006.01)　　*C07C 67/56* (2006.01)
*C07C 69/732* (2006.01)　　*C09K 15/08* (2006.01)

(86) International application number:
**PCT/JP2008/067528**

(87) International publication number:
**WO 2009/041640 (02.04.2009 Gazette 2009/14)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **26.09.2007　JP 2007249759**

(71) Applicant: **Takasago International Corporation Tokyo-to 144-8721 (JP)**

(72) Inventors:
• **TOYOHARA, Yoshikazu**
　**Kanagawa 254-0073 (JP)**
• **HIRAMOTO, Tadahiro**
　**Kanagawa 254-0073 (JP)**

(74) Representative: **Uchida, Kenji et al**
**S.A. Fedit-Loriot et Autres**
**38, avenue Hoche**
**75008 Paris (FR)**

(54) **PLANT EXTRACT AND USE THEREOF**

(57)　An object of the present invention is to provide a plant extract which comprises o-diphenol type chlorogenic acids in a high concentration. Also, it is to provide a plant extract excellent in deodorizing property or anti-oxidation property, which does not substantially comprise ferulylquinic acids and caffeine content. The present invention provides a plant extract, which comprises chlorogenic acids comprising an o-diphenol type chlorogenic acid and does not substantially comprise Ferulylquinic acid and caffeine, which is excellent in deodorizing property or anti-oxidation property.

EP 2 196 208 A1

**Description**

TECHNICAL FIELD

The present invention relates to a plant extract which comprises an o-diphenol type chlorogenic acid as one of the components that constitute a chlorogenic acid composition. Further, it relates to a plant extract which comprises an o-diphenol type chlorogenic acid in a large amount but does not substantially comprise ferulylquinic acid, and relates to a plant extract which comprises an o-diphenol type chlorogenic acid in a large amount but does not substantially comprise ferulylquinic acid and caffeine.

In addition, the invention relates to the use of these plant extracts.

BACKGROUND OF THE INVENTION

In recent years, accompanied by the diversification of life, improvement of living degree, change or improvement of consciousness and the like, it became a habit to pay attention to various points about us. One of them is the presence of various malodors. The main malodor components as the object include ammonia, urea, indole, skatole, amines and the like nitrogen compounds, methyl mercaptan, hydrogen sulfide, dimethyl sulfide and the like sulfur compounds, butyric acid and the like lower fatty acid and the like.

There are a large number of reports on the measures for eliminating or alleviating these malodors and the deodorants to be used for eliminating or alleviating the malodors.

For example, there is known a method for eliminating or alleviating malodors by allowing activated carbon to adsorb the components which cause malodor, but its effect cannot be said sufficient. In addition, there are reports on the use, as a deodorant, of a plant extract which comprises various chemical substances. For example, there are reports on the use, as a deodorant, of a Theaceae plant extract or an Apiaceae plant extract (Patent References 1 and 2). Though it can be said that these deodorants are deodorants which are friendly to the environment, it cannot be said that these are satisfactory from the viewpoint of deodorizing effect.

On the other hand, it has been known for a long time that qualities of organic substances such as oils and fats are changed by oxidation or heating and thereby undergo deterioration or deprivation of their originally possessed properties. In order to solve that point, a large number of antioxidants have been developed and reported. For example, synthetic antioxidants such as butylated hydroxytoluene (BHT) and butylhydroxyanisole (BHA) are broadly used as those which have excellent antioxidant property. However, though the antioxidants such as BHT and BHA are effective in view of their effects, various problems on these compounds have been pointed out from the viewpoint of safety. On the contrary, natural antioxidants such as ascorbic acid and tocopherol and natural antioxidants such as an extract from a bamboo (Patent Reference 3) do not have the problems of the aforementioned synthetic antioxidants in view of their safety, but instead, ascorbic acid has problems remained unsolved such as its markedly insoluble nature in fat-soluble substances, and tocopherol has problems remained unsolved such as the presence of peculiar malodor and off-taste and has a problem remained unsolved in that its preparation method is complex by the technique of Patent Reference 3.

It has been reported so far that coffee beans have various excellent effects including deodorizing effect and anti-oxidation effect, and a large number of methods have been reported on the preparation of extracts from coffee beans, which comprise chlorogenic acids considered as one of their active ingredients. However, when chlorogenic acid is extracted from coffee beans, caffeine is also extracted at the same time. It has been reported that caffeine does not exert good influence upon human, so that studies have been carried out on techniques for lessening the caffeine content of coffee bean extract.

For example, a technique has been reported for selectively removing caffeine in extracting chlorogenic acid from coffee beans using a supercritical fluid (Patent Reference 4). Though caffeine can be selectively removed by this technique, the device for extracting caffeine using a supercritical fluid is so expensive that there is an inconvenience in that considerable costs are required such as the costs for purchasing, managing and maintaining the device, the cost for purchasing the fluid and the operation cost at the time of extraction.

Contrary to this, it became reasonably possible to selectively remove caffeine with less cost by the use of synthetic resin adsorbent (Patent References 5, 6 and 7), but it cannot be said that the degree of removing caffeine is sufficient.

Patent Reference 1: JP-A-2006-102477
Patent Reference 2: JP-A-2003-306417
Patent Reference 3: JP-A-2006-116433
Patent Reference 4: JP-A-53-18772
Patent Reference 5: JP-A-4-145049
Patent Reference 6: JP-A-51-91368

Patent Reference 7: JP-A-2006-306799

DISCLOSURE OF THE INVENTION

PROBLEMS THAT THE INVENTION IS TO SOLVE

The present inventors already have reported on a deodorant which has excellent deodorizing property and uses raw coffee beans or the like as the raw material (JP-A-10-212221), and since then, have carried out studies with the aim of developing a deodorant which has further superior effect and, as a result, they have obtained knowledge that a substance which does not exerts good influence upon deodorizing effect seems to be coexisting in the components contained in extracts which use coffee beans or the like as the raw material, so that they have conducted various means for the purpose of specifying the substance which does not exerts good influence upon deodorizing effect. In addition, the inventors have also obtained knowledge during the process of these studies that the substance which does not exerts good influence upon deodorizing effect also does not exerts good influence upon anti-oxidation effect. Accordingly, a problem of the invention is to provide a composition having excellent deodorizing property or anti-oxidation property.

MEANS FOR SOLVING THE PROBLEMS

As a result of carrying out intensive studies with the aim of solving the aforementioned problem, the inventors have obtained the knowledge that a plant extract which is a chlorogenic acid composition that comprises o-diphenol type chlorogenic acids such as monocaffeoylquinic acid in a large amount, wherein ferulylquinic acid is not substantially present therein, is excellent in deodorizing property or anti-oxidation property. In addition, by further advancing the studies, knowledge was obtained that a plant extract which is a chlorogenic acid composition that comprises o-diphenol type chlorogenic acids such as monocaffeoylquinic acid in a large amount, wherein the caffeine content is markedly small, is excellent in deodorizing property or anti-oxidation property. By further conducting the studies based on such knowledge, the inventors have finally accomplished the invention.
That is, a gist of the invention is as described in the following.

[1] A plant extract which comprises an o-diphenol type chlorogenic acid and does not substantially comprise ferulylquinic acid.
[2] The plant extract according to [1], which does not substantially comprise caffeine.
[3] The plant extract according to [1] or [2], wherein a content of the o-diphenol type chlorogenic acid is 30% by mass or more based on a total amount of the plant extract.
[4] The plant extract according to any one of [1] to [3], wherein a total content of a polyphenols is 40% by mass or more based on a total amount of the plant extract.
[5] The plant extract according to any one of [1] to [4], which is an extract extracted from one species or two or more species of coffee beans selected from the group consisting of a raw coffee bean, a light roast coffee bean having an L value of 26 or more and a decaffeinated coffee bean.
[6] The plant extract according to any one of [1] to [5], which has a deodorizing property or anti-oxidation property.
[7] A product which comprises the plant extract according to any one of [1] to [6], wherein the product is selected from the group consisting of a fragrance product, a toiletry product, a food or drink, an oral care product and a pharmaceutical.
[8] A deodorant composition or antioxidant composition, which comprises the plant extract according to any one of [1] to [6].
[9] A product which comprises the deodorant composition or antioxidant composition according to [8], wherein the product is selected from the group consisting of a fragrance product, a toiletry product, a food or drink, an oral care product and a pharmaceutical.

ADVANTAGE OF THE INVENTION

By the invention, a plant extract which comprises chlorogenic acids comprising an o-diphenol type chlorogenic acid and does not substantially comprise ferulylquinic acid can be provided. In addition, a plant extract which comprises chlorogenic acids comprising an o-diphenol type chlorogenic acid in a high concentration can be provided. These plant extracts are excellent in deodorizing property or anti-oxidation property. What is more, since the caffeine amount contained in the plant extract of the invention is small, it is not necessary to worry about bad influence of caffeine upon human. Since the plant extract of the invention can be blended in a broad range of products and can provide excellent effect as well, this is a markedly practical invention.

BEST MODE FOR CARRYING OUT THE INVENTION

That is, the invention described in the above-mentioned [1] is a plant extract, which comprises chlorogenic acids comprising an o-diphenol type chlorogenic acid and does not substantially comprise ferulylquinic acid. In this case, the term "does not substantially comprise ferulylquinic acid" means that ferulylquinic acid cannot be detected by the present analytical methods. Since ferulylquinic acid can be detected by the present analytical methods when 0.3% by mass of ferulylquinic acid is contained in the plant extract, "does not substantially comprise ferulylquinic acid" also means that the content of ferulylquinic acid is less than 0.3% by mass. The invention described in the above-mentioned [1] is also a plant extract, which comprises chlorogenic acids substantially consisting of an o-diphenol type chlorogenic acid and does not substantially comprise ferulylquinic acid. In this case, the chlorogenic acids substantially consisting of an o-diphenol type chlorogenic acid mean chlorogenic acids which mostly comprise an o-diphenol type chlorogenic acid but may also comprise, in a small amount, chlorogenic acids other than ferulylquinic acid, and mean chlorogenic acids which may comprise the aforementioned other chlorogenic acids in a small amount with the proviso that the expected object of the invention can be attained.

The invention described in the above-mentioned [2] is one according to the invention described in [1], which does not substantially comprise caffeine. The case "does not substantially comprise caffeine" is the same as the case of the above-mentioned ferulylquinic acid. The term "does not substantially comprise caffeine" means that caffeine cannot be detected by the present analytical methods. Since caffeine can be detected by the present analytical methods when 0.1% by mass of caffeine is contained in the plant extract, "does not substantially comprise caffeine" also means that the content of caffeine is less than 0.1 % by mass.

The invention described in the above-mentioned [3] is one according to the above-mentioned [1] or [2], wherein a content of the o-diphenol type chlorogenic acid is 30% by mass or more based on a total amount of the plant extract. The invention described in the above-mentioned [4] is an invention according to the above-mentioned [1] to [3], wherein a total content of polyphenols is 40% by mass or more based on a total amount of the plant extract.

The invention described in the above-mentioned [5] is one according to any one of the above-mentioned [1] to [4], which is an extract extracted from one species or two or more species of coffee beans selected from the group consisting of a raw coffee bean, a light roast coffee bean having an L value of 26 or more and a decaffeinated coffee bean.

The invention described in the above-mentioned [6] is one according to any one of the above-mentioned [1] to [5], which has a deodorizing property or anti-oxidation property.

The invention described in the above-mentioned [7] is an invention on a product which comprises the plant extracts according to any one of the above-mentioned [1] to [6], wherein the product is selected from the group consisting of a fragrance product, a toiletry product, a food or drink, an oral care product and a pharmaceutical.

The invention described in the above-mentioned [8] is an invention on a deodorant composition or antioxidant composition which comprises the plant extracts according to any one of the above-mentioned [1] to [6].

The invention described in the above-mentioned [9] is a product which comprises the deodorant composition or antioxidant composition according to the above-mentioned [8], wherein the product is selected from the group consisting of a fragrance product, a toiletry product, a food or drink, an oral care product and a pharmaceutical.

The following describes the invention in detail.

The o-diphenol type chlorogenic acid as defined in the invention means a chlorogenic acid which has an o-diphenol structure. The o-diphenol structure means a structure in which hydroxyl groups are directly substituted on benzene rings and the hydroxyl groups are adjacent to each other. As illustrative examples of the o-diphenol type chlorogenic acid, monocaffeoly-quinic acids such as 3-caffeoylquinic acid (3-CQA), 4-caffeoylquinic acid (4-CQA) and 5-caffeoylquinic acid (5-CQA); dicaffeoylquinic acids such as 3,4-dicaffeoylquinic acid (3,4-CQA), 3,5-dicaffeoylquinic acid (3,5-CQA) and 4,5-dicaffeoyl-quinic acid (4,5-CQA), and the like can be cited.

In this connection, the above-mentioned o-diphenol type chlorogenic acid is contained in coffee beans, and ferulylquinic acid is also contained therein. As the monoferulylquinic acid, 3-ferulylquinic acid (3-FQA), 4-ferulylquinic acid (4-FQA), 5-ferulylquinic acid (5-FQA) and the like are known. One of the characteristics of the invention is that these ferulylquinic acids are substantially excluded.

In addition, the above-mentioned o-diphenol type chlorogenic acid is contained in coffee beans, and caffeine is also contained therein. One of the characteristics of the invention is that caffeine is substantially excluded.

In this connection, according to the invention, substances which are contaminated accompanied by the extraction operation of a plant may be present.

The content of the o-diphenol type chlorogenic acids as defined in the above-mentioned [3] of the invention means the content of the above-mentioned monocaffeoylquinic acid and dicaffeoylquinic acid. A plant extract in which the content of the aforementioned o-diphenol type chlorogenic acids is 30% by mass or more based on the total amount of the plant extract is particularly superior in the deodorizing property and anti-oxidation property. In addition, it is more desirable that the content of the o-diphenol type chlorogenic acids is from 30 to 50% by mass based on the total amount of the plant extract.

The polyphenols as defined in the above-mentioned [4] of the invention means a compound which has two or two or more phenolic hydroxyl groups. According to the invention, a glycoside of the aforementioned polyphenol is also included in the polyphenols. As the polyphenols of the invention, a polyphenol having the o-diphenol structure and a glycoside thereof are desirable. A plant extract in which all of the content of the aforementioned polyphenols which are present in the plant extract (to be referred sometimes to as the total content of polyphenols hereinafter) is 40% by mass or more based on the total amount of the plant extract is particularly superior in the deodorizing property and anti-oxidation property. In addition, it is more desirable that the total content of polyphenols is from 40 to 55% by mass based on the total amount of the plant extract.

The plant material for preparing the plant extract of the invention is not particularly limited with the proviso that it is a plant which comprises a chlorogenic acid, but a plant which comprises a chlorogenic acid in a large amount is desirable. As the plant which comprises a chlorogenic acid in a large amount, coffee beans, Ku Ding Tea, Mate tea (leaves), sunflower seeds, Artemisia and the like can be exemplified, though not limited thereto. According to the invention, coffee beans are desirable in view of the easiness to obtain the material and the like, and particularly, raw coffee beans, light roast coffee beans having an L value of 26 or more or decaffeinated coffee beans are desirable. The aforementioned plant may be directly subjected to an extraction treatment, or may be subjected to a pretreatment before carrying out the extraction treatment, such as drying, pulverization or the like of the plant, and then subjected to the extraction treatment.

Kinds and production areas of the coffee beans to be used as the material in the invention are not particularly limited, so that they may be any coffee beans or Coffea arabica, Coffea robusta, Coffea liberica and the like, and coffee beans of any production areas such as Brazilian products, Colombian products and the like can be used and beans of one species alone may be used or blended beans of two species or more may be used. In addition, since poor quality coffee beans or small grain coffee beans which are generally discarded as having no commercial value can also be used, effective use of coffee beans can be planned.

The L value to be used in the invention is known as an index which represents a roasting degree of coffee beans and is a value obtained by measuring lightness of a pulverized product of roasted coffee beans by a color difference meter. The "L value" by color specification (JIS Z 8730) is lightness wherein black is represented by an L value of 0, and white by an L value of 100. When coffee roast beans are shown by the L value, it is known that values of the L value are slightly changed depending on the measuring conditions of the L value. Regarding L value of coffee roast beans generally used for drinking, generally in Japan, roast beans having an L value of from 26 to 30 are called light roast beans, and roast beans having an L value of from 18 to 26 medium roast beans and roast beans having an L value of from 14 to 18 deep roast beans. According to the invention, desirable results can be obtained when roast beans of light roast are used. This means that desirable results can also be obtained when roast beans of light roast are used.

The "decaffeinated coffee beans" according to the invention generally means coffee beans to which a treatment of extracting and removing caffeine from coffee beans is applied by a conventional method. As the method for treating decaffeination from coffee beans, a method in which caffeine is extracted from raw coffee beans using water, methylene chloride or supercritical carbon dioxide as the solvent can be cited. According to the invention, beans prepared by roasting the above-mentioned "decaffeinated coffee beans" by an extremely light roasting are also to be included in the "decaffeinated coffee beans". In this connection, the extremely light roasting of coffee beans means that the roasting treatment is carried out for a roasting time of less than 3 minutes using a roaster generally used by those skilled in the art. As the "decaffeinated coffee beans" of the invention, any coffee beans after a caffeine removing treatment can be used without particular limitation. When coffee beans from which caffeine was removed as many as possible are used, working efficiency of the caffeine removal by this method is increased in proportion thereto.

As one of the characteristics, the plant extract of the invention has deodorizing property or anti-oxidation property. As a desirable method for preparing the plant extract of the invention, for example, there can be cited a method in which the aforementioned plant material is subjected to an extraction treatment using water or a hydrous water miscible organic solvent and then the extract is treated with a synthetic resin adsorbent.

Regarding the water miscible organic solvent as the aforementioned extraction solvent, lower alcohols such as methanol, ethanol or 2-propanol and ketones such as acetone or methyl ethyl ketone are desirable, though not limited thereto. Regarding the hydrous water miscible organic solvent as the aforementioned extraction solvent, it is desirable to use a mixture of 90% or less of water and 10% or more of the aforementioned water miscible organic solvent (volume ratio). According to the invention, an aqueous solution comprising from 10 to 80% by volume of ethanol is particularly desirable, and an aqueous solution comprising from 20 to 60% by volume of ethanol is more desirable.

Since extraction conditions such as extraction time greatly vary depending on the kinds and properties of the plant material to be used, the extraction solvent to be used and the like, optimum extraction conditions may be set depending on these factors and therefore cannot be defined in a wholesale manner. In this connection, the temperature at the time of extraction is not particularly limited, but it is advantageous to set it to approximately reflux temperature of the solvent to be used. In addition, the extraction time cannot also be defined in a wholesale manner, but for example, approximately 1 hour or more is desirable.

As the aforementioned synthetic resin adsorbent, resin-based adsorbents such as an aromatic-based resin, a methacrylic acid ester-based resin and an acrylonitrile aliphatic-based resin can be used. Among them, an adsorbent of aromatic-based resin such as a styrene-divinylbenzene-based synthetic resin is desirable, and aromatic-based resin such as a no substituent group type styrene-divinylbenzene-based synthetic resin is particularly desirable. Desirable synthetic resin adsorbents are on the market and can be obtained easily. For example, Diaion HP-10, HP-20, HP-21, HP-30, HP-40, SP-205, SP-206, SP-207, SP-800, SP-850 and SP-875 (trade names, mfd. by Mitsubishi Chemical Corporation) and Amberlite XAD-2, XAD-4, XAD-16 and XAD-2000 (trade names, mfd. by Rohm & Haas Co.) can be cited.

According to the invention, it is important to treat the aforementioned solvent extract by contacting with a synthetic resin adsorbent, and it is advantageous to carry out the contact treatment with the synthetic resin adsorbent after evaporating most of the solvent from the aforementioned solvent extract and subsequent dilution with water. The aforementioned evaporation of most of the solvent indicates that most of the solvent contained in the solvent extract is evaporated from the extract, which means timing when specific gravity of the liquid after evaporation of the solvent is 1.06 or more.

Degree of the dilution with water after evaporation of almost all portion of the solvent is such that the Brix value becomes preferably from about 3 to 50, more preferably from about 3 to 20, and further suitable result can be obtained when it became approximately from 5 to 10. In this connection, the aforementioned Brix value means a scale of the displayed measured value when measured using a Brix meter (refractometer). Brix meter is one of the instruments for analysis which uses reflection phenomenon of light and shows the soluble solid content in an aqueous solution making use of the reflection phenomenon of light. Accordingly, when an aqueous solution has a Brix value of 10, concentration of the water-soluble solid content in the aqueous solution is 10%.

Regarding the contact treatment method, a desirable result can be obtained by the use of a column method in which the aforementioned synthetic resin adsorbent is packed in a column and the aforementioned solvent extract is poured into said column.

Since the conditions for treating the aforementioned solvent extract by contacting with a synthetic resin adsorbent vary depending on the kind and property of the plant material to be used, kind and property of the solvent extract to be used and the like, optimum extraction conditions may be set depending on these factors and therefore cannot be defined in a wholesale manner, but when a column method is employed for example, these can be selected from the following contact treatment conditions. There can be cited a contact treatment condition in which contact treatment temperature is set to 60°C or less, amount of the adsorbent to be packed in a column is set to 3 times (by mass) or more of the solid content of solvent extract and space velocity (SV value), which means velocity of the solvent extract passing through the column, is set to 2 or less. In addition, according to the invention, setting of the frequency of column treatment to two or more times, washing of the synthetic resin after contact treatment with water (e.g., approximately from 1 to 3 times by volume of the amount of the resin) or the like may be effective depending on the extraction conditions to be employed, because those which are qualitatively excellent can be obtained, for example, o-diphenol can be recovered efficiently and the like.

The invention also relates to a deodorant composition or antioxidant composition which comprises the above-mentioned plant extract. In addition, the invention also relates to a deodorant composition or antioxidant composition which comprises the above-mentioned chlorogenic acid. In these compositions, the o-diphenol type chlorogenic acid is present as the active ingredient which realizes the deodorizing function or anti-oxidation function.

A conventionally known plant extract may be contained in the above-mentioned deodorant composition or antioxidant composition, with the proviso that it does not spoil the desired object.

A flavoring or fragrance can be added to the plant extract defined by the invention or the deodorant composition or antioxidant composition which comprises the above-mentioned chlorogenic acid or plant extract.

As the flavoring, synthesized aromatic materials such as esters, alcohols, aldehydes, ketones, acetals, phenols, ethers, lactones, furans, hydrocarbons and acids, and natural origin aromatic materials and the like, can be cited.

As the flagrance, synthesized aromatic materials such as hydrocarbons, alcohols, phenols, aldehydes and/or acetals, ketones and/or ketals, ethers, synthetic musks, acids, lactones, esters and halogen-containing compounds, and natural origin aromatic materials and the like, can be cited.

Further, in addition to the above-mentioned flavorings and flagrances, the aroma chemicals described in "Investigation on the Actual Using State of Food Flagrance Compounds in Japan (written in Japanese)" (Welfare Science Research Report (written in Japanese), 2000; published in 2001 by Japan Flavor and Fragrance Material Association), "Synthesized Flavoring, Chemistry and Merchandise Information (written in Japanese" (published on March 6, 1996, edited by G. Indo, The Chemical Daily Co., Ltd.), "Perfume and Flavor Chemicals (Aroma Chemicals) 1, 2" (Steffen Arctender (1969) and the like, also can be used.

These flavorings and flagrances may be used by mixing one species or two or more species.

Those on the market can also be used. In addition, single article may be used as a synthesized product or may be prepared from a natural origin such as plants. Essential oil, resinoid, balsam, absolute, concrete, tincture and the like can also be prepared by conventionally known methods.

It is advantageous to set blending amount of the flavoring or fragrance to from 10,000 to 0.0001 times by mass based on the total amount of the plant extract or chlorogenic acid composition.

The plant extract or chlorogenic acid composition according to the invention can be used as such or can be added as such directly to food or the like. In addition, it is possible to dissolve or disperse it in an appropriate liquid carrier (e.g., ethanol, ethanol aqueous solution, benzyl alcohol, middle chain fatty acid triglyceride (MCT) and the like) or mix with an appropriate powder carrier (e.g., polysaccharides, chemically modified starch, activated carbon, silica gel and the like) or adsorb thereto. Also, depending on circumstances, it is possible to make it into pharmaceutical preparations such as emulsions, water dispersible powders, powders, tablets and the like, by adding an emulsifying agent, a dispersant, a suspending agent, a spreading agent, a penetrating agent, a wetting agent or a stabilizing agent.

In addition, various compounding agents may be added to the plant extract or composition of the invention. As such additive agents, it is possible also to blend compounding agents such as an extender, an antioxidant, a pigment, an antiseptic or antibacterial agent, a deodorant, a moisture absorbent, a surfactant, a carrier, a pH adjustor, a sweetener, a milk component, an acidulant or a nutrition enriching agent, alone or in combination of two or more species thereof. Blending amount of the above-mentioned compounding agents is not particularly limited with the proviso that it is an amount which can achieve the object of the invention.

As the extender, saccharides, polysaccharides, chemically modified starch, casein, gelatin, carboxymethylcellulose (CMC), lecithin and the like can be used.

As the antioxidant, butyl hydroxytoluene, butyl hydroxyanisole, citric acid, bioflavoic acid, glutathione, selenium, lycopene, vitamin A, vitamin E, vitamin C and the like, as well as enzymes having anti-oxidation property such as free radical scavengers obtained from pyrrolopyrrole derivatives or from extracts of various plants, superoxide dismutase and glutathione peroxidase, and the like, can be used.

As the pigment, organic synthetic pigments (tar pigments) such as a dyestuff, a lake and an organic pigment, natural pigments and the like are known, and illustratively, hibiscus color, huckleberry color, plum color, seaweed color, dewberry color, grape juice color, blackberry color, blueberry color, mulberry color, morello cherry color, red currant color, loganberry color, paprika powder, malt extract, rutin, flavonoid, red cabbage color, red radish color, *adzuki* bean color, turmeric color, olive tea, cowberry color, *Chlorella* powder, saffron color, beefsteak color, strawberry color, chicory color, pecan nut color, monascus color, safflower color, purple sweet pupate color, lac color, spirulina color, onion color, tamarind color, red pepper color, gardenia color, *shikon* color, red sandal wood color, krill color, orange color, carrot carotene, caramel, titanium dioxide, sodium iron chlorophyllin, riboflavin, norbixin potassium, norbixin sodium, amaranth, erythrosine, new coccine, phloxine B, rose Bengal, acid red, coothrazine, sunset yellow, fast green, brilliant blue, indigocarmine, lake red C, lithol red, rhodamine, phloxine, indigo, ponceau, orange I, sudan blue, mica, talc, calcium carbonate, kaolin, silicic anhydride, aluminum oxide, rouge, iron oxide, ultramarine, carbon black, titanium dioxide, zinc oxide, mica, bismuth oxychloride, boron nitride, photochromic pigment, fine grain composite powder (hybrid fine powder), synthetic mica and the like can be used.

As the antiseptic or antibacterial agent, benzoic acid, sodium benzoate, isopropyl p-hydroxybenzoate, isobutyl p-hydroxybenzoate, ethyl p-hydroxybenzoate, methyl p-hydroxybenzoate, butyl p-hydroxybenzoate, propyl p-hydroxybenzoate, sodium sulfite, sodium hyposulfite, potassium pyrosulfite, sorbic acid, potassium sorbate, sodium dehydroacetate, thujaplicin, *Aralia cordata* extract, *Styrax japonica* extract, *Artemisia capillaris* extract, Oolong tea extract, soft roe protein extract, enzyme-digested pearl barley extract pectin digest, chitosan, lysozyme, ε-polylysine and the like can be used, and tea catechins, apple polyphenol and the like can be used.

As the deodorant, for example, a deodorants by chemical reaction action (an acidifying agent, an alkalifying agent, an oxidizing agent, a reducing agent and the like), a deodorant by addition and condensation action (addition agent: (meth)acrylic acid ester, maleic acid ester and the like, condensing agent: glyoxal and the like), a deodorant by adsorption action (porous adsorbent such as a mixture of neutral activated carbon or activated carbon with a chemical reaction agent, an attenuation carbon adsorbent, zeolite or activated clay, and the like), a deodorant by enzyme action, a polyphenol type deodorant (persimmon polyphenol, tea catechin, rosemary extract, Oolong tea extract, mugwort extract, *Quercus stenophylla* leaf extract, rice bran or soybean roast extract and the like), cyclodextrin, champignon extract, rooibos extract, sodium iron chlorophyllin and the like can be cited.

As the moisture absorbent, calcium chloride, a high water absorption polymer and the like can be cited.

As the surfactant, it is desirable to use nonionic type surfactants, particularly a polyoxyethylene alkyl ether, a fatty acid alkylolamide or an acyl glutamic acid, alone or in combination of two or more species. As examples of the polyoxyethylene alkyl ether, polyoxyethylene stearyl, polyoxyethylene hydrogenated castor oil and the like can be cited. As an example of the fatty acid alkylolamide, coconut oil fatty acid diethanolamide can be cited. As the acyl glutamic acid type, glutamic acid esters of saturated and unsaturated fatty acids having from 12 to 18 carbon atoms, and as their mixtures, coconut oil fatty acid, hydrogenated coconut oil fatty acid, palm oil fatty acid, hydrogenated palm oil fatty acid, beef tallow fatty acid, hydrogenated beef tallow fatty acid and the like, can be cited, and illustratively, N-coconut oil fatty acid acyl-L-glutamic acid triethanolamine, lauroyl-L-glutamic acid triethanolamine, sodium N-coconut oil fatty acid acyl-L-glutamate, sodium N-lauroyl-L-glutamate, sodium N-myristoyl-L-glutamate, sodium N-coconut oil fatty acid beef tallow fatty acid acyl-L-glutamate, potassium N-coconut oil fatty acid acyl-L-glutamate and the like can be used.

As the carrier, gum arabic, dextrin and the like can be used.

As the pH adjustor, adipic acid, citric acid (anhydrous), glucono-δ-lactone, potassium gluconate, succinic acid, disodium succinate, sodium acetate (anhydrous), L-tartaric acid, potassium hydrogen L-tartarate, sodium L-tartarate, sodium bicarbonate, sodium carbonate (anhydrous), lactic acid, glacial acetic acid, fumaric acid, DL-malic acid, phosphoric acid, potassium dihydrogenphosphate, disodium hydrogenphosphate (anhydrous), sodium dihydrogenphosphate (anhydrous), citric acid (crystalline), sodium citrate, gluconic acid, sodium gluconate, monosodium succinate, sodium acetate (crystalline), DL-tartaric acid, potassium hydrogen DL-tartarate, sodium DL-tartarate, potassium carbonate (anhydrous), sodium carbonate (crystalline), carbon dioxide, sodium lactate, disodium dihydrogen-pyrophosphate, monosodium fumarate, sodium DL-malate, dipotassium hydrogenphosphate, disodium hydrogen-phosphate (crystalline), sodium dihydrogenphosphate (crystalline), itaconic acid, α-ketoglutaric acid (extract), phytic acid and the like can be used.

As the sweetener, sucrose, fructose, lactose, cane sugar, glucose, palatinose, maltose, trehalose, sorbitol, erythritol, maltitol, reduced palatinose, xylitol, lactitol, starch syrup, oligosaccharide, aspartame, sucralose, acesulfam K, saccharin, stevia, neotame, alitame, thaumatin, neohesperidin dihydrochalcone, licorice and the like can be used.

As the acidulant, acetic acid, lactic acid, citric acid and the like can be used.

As the milk component, raw milk, milk, whole milk powder, skim milk powder, fresh cream and the like can be used.

As the nutrition enriching agent, vitamins, various amino acids, minerals such as mineral calciums, irons, zincs, coppers, and the like can be used.

Regarding the objects to which the plant extract or composition of the invention can be added, any material can be blended therewith with the proviso that characteristics of the plant extract or composition of the invention can be exerted. In illustratively describing, a fragrance product, a toiletry product, a foundation cosmetic, a hair cosmetic, a bath preparation, a body care product, a cleanser, a finishing agent, an aromatic deodorant, a food or drink, an oral care product, a pharmaceutical and the like can be cited, though not limited thereto.

That is, the invention relates also to a fragrance product, a toiletry product, a food or drink, an oral care product and a pharmaceutical, which comprise the plant extract, deodorant composition or antioxidant composition described in the above. In addition, the invention also relates to a deodorizing method or anti-oxidation method, wherein the plant extract described in the above is added to a product selected from a fragrance product, a toiletry product, a food or drink, an oral care product and a pharmaceutical.

As the fragrance product, perfume, eau de toilette, eau de cologne, shower cologne and the like can be cited. As the foundation cosmetic, skin milk, cleansing cream, face lotion, after shave lotion, foundation, rouge, talcum powder and the like can be cited.

As the hair cosmetic, shampoo preparations such as shampoo, rinse, conditioner, rinse in shampoo and treatment, hair dressings such as pomade, hair tonic, hair liquid and hair jell , revitalizing hair tonic, hair dye, cold wave and the like can be cited.

As the toiletry product, toilet soap, bath soap, transparent soap and the like can be cited.

As the cleanser, powder cleanser for clothes, liquid cleanser for clothes, soft finish preparation, kitchen cleanser, toilet cleanser, bath room cleanser, glass cleaner, mildew remover and the like can be cited.

As the aromatic deodorant, gel aromatic deodorant, mist type aromatic deodorant, impregnation type aerosol aromatic deodorant and the like can be cited.

As the food or drink, for example, drinks such as drink containing fruit juice, drink without fruit juice, tea type drink and powder drink, ices such as ice cream, sherbet and ice cake, desserts such as pudding, jelly, Bavarian cream and yogurt, milk products such as raw milk, milk, whole milk powder, skim milk powder, fresh cream, concentrated milk, skim milk, partially skimmed milk and condensed milk, confectionaries such as cookie, chocolate, gum and candy, fish paste products and the like can be cited.

As the pharmaceutical, tablets, liquid drugs, capsule type drugs, granular drugs and the like can be cited. As the oral care product, mouth washer, tooth powder, oral care gum, oral care candy and the like can be cited.

Amount of the plant extract or chlorogenic acid composition of the invention to be added to various types of fragrance or cosmetic, food or drink, oral care product or pharmaceutical sharply varies depending on the object matter and the like, but it is desirable to set it to generally from 0.000001% by mass to 50% by mass based on the object matter, and it is more desirable to set it to from 0.00001% by mass to 10% by mass.

The invention also includes the following.

(1) A method for producing a plant extract which comprises chlorogenic acids comprising an o-diphenol type chlorogenic acid and substantially does not comprise ferulylquinic acid and caffeine, wherein the method comprises at least a step of subjecting a plant comprising a chlorogenic acid to an extraction treatment using water or a hydrous water miscible organic solvent, a step of subjecting the aforementioned extraction treated matter to a contact treatment with a synthetic resin adsorbent, and a step of concentrating the unadsorbed solution on synthetic resin adsorbent obtained by the aforementioned contact treatment step.

(2) The method for producing a plant extract according to the above-mentioned (1), wherein the step of subjecting the extraction treated matter to a contact treatment with a synthetic resin adsorbent is a step of subjecting the extraction treated matter to a contact treatment with a synthetic resin adsorbent using a column method.

(3) The method for producing a plant extract according to the above-mentioned (2), which further comprises a step of removing the extraction solvent from the extract obtained by the extraction treatment step and a step of diluting the aforementioned solvent-removed matter again with the solvent.

(4) The method for producing a plant extract according to the above-mentioned (2) or (3), wherein a specific gravity of the solvent-removed matter is 1.06 or more and the solvent-removed matter is diluted with the solvent to have a Brix value of from 3 to 50.

(5) The method for producing a plant extract according to any one of the above-mentioned (2) to (4), wherein, in the step of subjecting the extraction treated matter to a contact treatment with a synthetic resin adsorbent using a column method, the contact treatment temperature is 60°C or less, amount of the adsorbent to be packed in the column is 3 times (by mass) or more based on a solid content of the solvent extract and space velocity (SV value) of the solvent extract passing through the column is 2 or less.

EXAMPLES

The following illustratively describes the invention based on Examples, but the invention is not limited to these Examples.

In this connection, measuring method of caffeoylquinic acid, measuring method of ferulylquinic acid, measuring method of caffeine and measuring method of total polyphenols are as follows.

Measuring method of the caffeoylquinic acids content

Carried out by a high performance liquid chromatography method (HPLC method).
Column: Cosmosil 5C18AR-II (4.6 x 150 mm, mfd. by Nacalai Tesque)
Solvent: A acetonitrile, B 0.01 M phosphoric acid aqueous solution
Flow rate: 0.8 ml/min
Time required for analysis: 30 minutes
0 to 15 minutes: a mixed solvent of A 9% and B 91 % is used (isocratic)
15 to 30 minutes: the sum of A and B is 100%, and make a gradient of from 9 to 100% for A and from 91 to 0% for B (gradient)
Measuring wavelength: 325 nm
Detector: ultraviolet ray absorptiometer (mfd. by Agilent Technologies)

Measuring method of ferulylquinic acid

Carried out by a high performance liquid chromatography method (HPLC method), in accordance with the method of Analyst, 109, 263 - 266, 1984.

Illustrative conditions are as follows.
Column: Spherisorb-ODS 2 (4.6 x 250 mm, mfd. by Waters)
Solvent: A methanol, B 0.01 M citrate buffer (pH 2.5)
Flow rate: 1 ml/min
Time required for analysis: 45 minutes
0 to 45 minutes: the sum of A and B is 100%, and make a gradient of from 20 to 70% for A and from 80 to 30% for B (gradient)
Measuring wavelength: 325 nm
Detector: ultraviolet ray absorptiometer (mfd. by Agilent)

Measuring method of caffeine

Carried out by a high performance liquid chromatography method (HPLC method). Mostly the same as the measuring method of caffeoylquinic acids content. However, the measuring wavelength is 280 nm.

Measuring method of total polyphenols,

Carried out by an absorbance method. The wavelength employed is 765 nm. Illustrative method
A 100 µl portion of a sample aqueous solution (concentration is 2 µg/µL), 7.5 ml of water and 300 µL of two times diluted aqueous solution of phenol reagent (acidity 1.8 N, mfd. by Nakalai Tesque) are put into a test tube and stirred, and then 1 mL of 20% sodium carbonate aqueous solution and 1.1 mL of water are further added thereto, followed by stirring. After allowing it to stand at room temperature for 1 hour, the amount of total polyphenols is known from the absorbance at 765 nm using a calibration curve (prepared using 5-chlorogenic acid (mfd. by Tokyo Chemical Industry)).

Example 1 Preparation of plant extract comprising chlorogenic acids comprising an o-diphenol type chlorogenic acid, from raw coffee beans

1a (Preparation of raw coffee bean extract)

To 900 g of a pulverized product of raw coffee beans (*Coffea robusta,* Indonesia) was added 60% hydrated alcohol in an amount of about 6 times by mass based on the pulverized product, and reflux extraction was carried out for 2 hours.
After concentration of a filtrate of the extract by an evaporator to dryness, water was added thereto to obtain 3370 g of a raw coffee bean extract having a Brix value of 5.
In this case, the Brix value was measured using a digital Brix meter PR-1 (mfd. by Atago) (the same shall apply hereinafter).

1b (Contact treatment of raw coffee bean extract)

Contact treatment of the above-mentioned extract was carried out at an SV value = 1 using a column filled with 5 times by mass (680 g) of a synthetic resin adsorbent (Diaion HP 20: mfd. by Mitsubishi Chemical Corporation) based on the solid content in the extract, and the unadsorbed solution on the resin from the column was received in a container. Next, 3 times by volume of water based on the resin was poured into the column, and the unadsorbed solution on the resin from the column was received in a container. The unadsorbed solution on the resin in the containers were combined and concentrated to have a Brix value of 5.
The resin was washed by passing 3 times by volume of 80% ethanol (2700 g) based on the resin through the column at an SV value = 1.5. Next, after removing ethanol by washing with water, the previously concentrated liquid was again subjected to a contact treatment in the same manner using this washed resin column. The unadsorbed solution on the resin were combined (5790 g) and the solvent was evaporated to obtain 71 g of a plant extract which comprises chlorogenic acids comprising an o-diphenol type chlorogenic acid (yield: 7.9% based on raw beans).
The contents of caffeoylquinic acids (CQAs), ferulylquinic acids (FQAs), total polyphenols and caffeine in the plant extract were measured. The measuring results are shown in Table 1.
Table 1 Extract analysis values

| | CQAs | FQAs | Total polyphenols | Caffeine |
|---|---|---|---|---|
| Content ratio (based on extract) | 36.2% by mass | Outside the detection limit | 43% by mass | Outside the detection limit |

CQAs: total amount of 3-CQA, 4-CQA, 5-CQA, 3,4-CQA, 3,5-CQA and 4,5-CQA (the same shall apply hereinafter)
FQAs: total amount of 3-FQA, 4-FQA, 5-FQA (the same shall apply hereinafter) Ferulylquinic acids were outside the detection limit (less than 0.3% by mass), and caffeine was outside the detection limit (less than 0.1 % by mass).

Test method 1 Deodorizing activity test (1)

Tyrosinase (mfd. by SIGMA, 1 mg) or laccase (mfd. by Amano Enzyme, 1 mg) was added as a polyphenol oxidase to each (2 mg) of 5-CQA (mfd. by Tokyo Chemical Industry), ferulic acid (mfd. by Tokyo Chemical Industry) and ferulylquinic acid and dissolved therein, and then sodium methyl mercaptan (mfd. by Tokyo Chemical Industry, 15% aqueous solution) (2 µl) was added thereto as a malodor substance and sealed therein, followed by stirring at room temperature for 10 minutes. The amount of methyl mercaptan in the head space part was measured using a gas detection tube (GASTEC Corporation, #71H) and the deodorizing ratio was calculated in accordance with the following mathematical expression.

The results are shown in Table 2. In this connection, a system to which each of CQA or FQA (2 mg) and polyphenol oxidase were not added was used as a control plot.

$$\text{Deodorizing ratio (\%)} = 100 - (\text{methyl mercaptan concentration of sample plot}/$$

$$\text{methyl mercaptan concentration of control plot}) \times 100$$

Table 2 Deodorizing ratio (%)

|  | 5-CQA | Ferulic acid | Ferulylquinic acid |
|---|---|---|---|
| Tyrosinase | 75 | -10 | -20 |
| Laccase | 100 | -18 | -18 |

In this case, the aforementioned ferulylquinic acid was prepared from raw coffee beans in accordance with the method of Analyst, 109, 263 - 266, 1984. Ferulylquinic acids (4.2 g) was obtained from raw coffee beans (1 kg).

Deodorizing activity test (2)

Deodorizing activity test was carried out on both of the raw coffee bean extract of the above-mentioned Example 1a (to be referred sometimes to as raw coffee bean extract solution) and the contact treated product of the raw coffee bean extract of the above-mentioned 1b (to be referred sometimes to as contact treated product).

That is, each of the raw coffee bean extract of the above-mentioned Example 1 a and contact treated product of 1b was weighed such that the total content of the polyphenol became 2 mg, laccase (mfd. by Amano Enzyme, 1 mg) was added thereto and dissolved therein and then sodium methyl mercaptan (mfd. by Tokyo Chemical Industry, 15% aqueous solution) (2 μl) was added thereto as a malodor substance, and thereafter, deodorizing activity test was carried out by carrying out the subsequent operations in the same manner as in the above-mentioned test method 1.

The measured results are shown in Table 3. In this connection, a system to which each extract and laccase were not added was used as a control plot.

Table 3

|  | Deodorizing ratio (%) | Composition (%) | Remarks |
|---|---|---|---|
| Raw coffee been extract | 30 | CQAs; 25.9<br>FQAs; 6.3<br>Caffeine; 10.0 | Corresponds to Comparative Example |
| Contact treated product | 100 | CQAs; 36.2<br>FQAs; outside the detection limit<br>Caffeine; outside the detection limit | Corresponds to Example |

Test method 2 Anti-oxidation activity test

As the anti-oxidation activity, to what extent it can eliminate DPPH (1,1-diphenyl-2-picrylhydrazyl) (mfd. by Tokyo Chemical Industry) radicals was evaluated by an absorptiometric method (520 nm). Its illustrative method is as the following anti-oxidation activity measuring method.

Used as the standard of radical elimination ability was Trolox (218940050: mfd. by ACROS ORGANICS) prepared from α-tocopherol which is a typical antioxidant substance by releasing its side chain to provide water-soluble nature. By preparing a calibration curve of Trolox concentration and absorbance (y = -0.2099x + 0.4179), anti-oxidation activity of each sample was evaluated by converting it into Trolox equivalent amount. The evaluation results are shown in Table 4. Regarding samples in the table, 5-CQA is manufactured by Tokyo Chemical Industry and ferulic acid is manufactured by Tokyo Chemical Industry.

Table 4

| Sample | Anti-oxidation activity (T. $\mu$mol/$\mu$mol) |
|---|---|
| Ferulic acid | 0.37 |
| Ferulylquinic acid | 0.35 |
| 5-CQA | 1.03 |
| Raw coffee bean extract (Example 1a) | 0.57 |
| Contact treated product (Example 1b) | 0.97 |

(Method for measuring anti-oxidation activity)

Method for measuring DPPH radical elimination ability of samples

1 mM ethanol solution of DPPH is prepared (39.4 mg/100 ml). After dissolution, DPPH is immediately used in the test (preparation when used).
By dissolving each sample in 50% v/v ethanol, its 100 mg/l solution is prepared.
A 4 ml portion of the above-mentioned sample solution and 1 ml of the DPPH solution are added to a test tube, followed by stirring and allowing to stand still for 30 minutes, and then absorbance at a wavelength of 520 nm is measured.
By applying absorbance of the sample to the calibration curve, Trolox equivalent amount (mol) is calculated.

Example 2 Preparation of plant extract comprising chlorogenic acids comprising an o-diphenol type chlorogenic acid, from light roast coffee beans

1 a (Preparation of light roast coffee bean extract)

Raw coffee beans (*Coffea robusta,* Indonesia) was lightly roasted to have an L value of 30, and to 500 g of a pulverized product of this roasted beans was added 50% hydrated alcohol in an amount of about 6 times by mass based on the pulverized product, and reflux extraction was carried out for 2 hours. After concentration of a filtrate of the extract by an evaporator to dryness, water was added thereto to obtain 1900 g of a raw coffee bean extract solution having a Brix value of 5.

1b (Contact treatment of light roast coffee bean extract)

Contact treatment of the above-mentioned extract was carried out at an SV value = 1 using a column filled with 5 times by mass (380 g) of a synthetic resin adsorbent (Diaion HP 20: mfd. by Mitsubishi Chemical Corporation) based on the solid content in the extract, and the unadsorbed solution on the resin from the column was received in a container. Next, 3 times by volume of water based on the resin was poured into the column, and the unadsorbed solution on the resin from the column was received in a container. The unadsorbed solution on the resin in the containers were combined and concentrated to have a Brix value of 5.
The resin was washed by passing 3 times by volume of 80% ethanol (1500 g) based on the resin through the column at an SV value = 2. Next, after removing ethanol by washing with water, the previously concentrated liquid was again subjected to a contact treatment in the same manner using this washed resin column. The unadsorbed solution on the resin were combined (2980 g) and the solvent was evaporated to obtain 38 g of a plant extract which comprises chlorogenic acids comprising an o-diphenol type chlorogenic acid (yield: 7.6% based on raw beans).
The contents of caffeoylquinic acids (CQAs), ferulylquinic acids (FQAs), total polyphenols and caffeine in the plant extract were measured. The measuring results are shown in Table 5.
Table 5 Extract analysis values:

| | CQAs | FQAs | Total polyphenols | Caffeine |
|---|---|---|---|---|
| Content ratio (based on the extract) | 34.9% by mass | Outside the detection limit | 41.2% by mass | Outside the detection limit |

While the invention has been described in detail and with reference to specific embodiments thereof, it will be apparent to one skilled in the art that various changes and modifications can be made therein without departing from the spirit and scope of the invention. This application is based on a Japanese patent application filed on September 26, 2007 (Japanese Patent Application No. 2007-249759), the entire contents thereof being thereby incorporated by reference.

INDUSTRIAL APPLICABILITY

By the invention, a plant extract which comprises chlorogenic acids comprising an o-diphenol type chlorogenic acid and does not substantially comprise ferulylquinic acid can be provided. In addition, a plant extract which comprises chlorogenic acids comprising an o-diphenol type chlorogenic acid in a high concentration can be provided. These plant extracts are excellent in deodorizing property or anti-oxidation property. What is more, since the caffeine amount contained in the plant extract of the invention is small, it is not necessary to worry about bad influence of caffeine upon human. Since the plant extract of the invention can be blended in a broad range of products and can provide excellent effect as well, this is a markedly practical invention.

**Claims**

1.   A plant extract, which comprises an o-diphenol type chlorogenic acid, and does not substantially comprise ferulylquinic acid.

2.   The plant extract according to claim 1, which does not substantially comprise caffeine.

3.   The plant extract according to claim 1 or 2, wherein a content of the o-diphenol type chlorogenic acid is 30% by mass or more based on a total amount of the plant extract.

4.   The plant extract according to any one of claims 1 to 3, wherein a total content of polyphenols is 40% by mass or more based on a total amount of the plant extract.

5.   The plant extract according to any one of claims 1 to 4, which is an extract extracted from one or two or more species of coffee beans selected from the group consisting of a raw coffee bean, a light roast coffee bean having an L value of 26 or more and a decaffeinated coffee bean.

6.   The plant extract according to any one of claims 1 to 5, which has a deodorizing property or anti-oxidation property.

7.   A product which comprises the plant extract according to any one of claims 1 to 6, wherein the product is selected from the group consisting of a fragrance product, a toiletry product, a food or drink, an oral care product and a pharmaceutical.

8.   A deodorant composition or antioxidant composition, which comprises the plant extract according to any one of claims 1 to 6.

9.   A product which comprises the deodorant composition or antioxidant composition according to claim 8, wherein the product is selected from the group consisting of a fragrance product, a toiletry product, a food or drink, an oral care product and a pharmaceutical.

## INTERNATIONAL SEARCH REPORT

| | International application No. |
|---|---|
| | PCT/JP2008/067528 |

**A. CLASSIFICATION OF SUBJECT MATTER**
A61K36/18(2006.01)i, A61K8/368(2006.01)i, A61K8/97(2006.01)i,
A61K31/191(2006.01)i, A61P39/06(2006.01)i, A61Q11/00(2006.01)i,
A61Q13/00(2006.01)i, A61Q15/00(2006.01)i, A61Q17/00(2006.01)i,
According to International Patent Classification (IPC) or to both national classification and IPC

**B. FIELDS SEARCHED**

Minimum documentation searched (classification system followed by classification symbols)
A61K36/18, A61K8/368, A61K8/97, A61K31/191, A61P39/06, A61Q11/00,
A61Q13/00, A61Q15/00, A61Q17/00, C07C67/56, C07C69/732, C09K15/08

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)
BIOSIS/MEDLINE/WPIDS(STN), CAplus(STN), JSTPlus(JDreamII),
JMEDPlus(JDreamII), JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2006-342145 A  (Oryza Oil & Fat Chemical Co., Ltd.),<br>21 December, 2006 (21.12.06),<br>Par. No. [0045]; table 1(examples 3, 4)<br>(Family: none) | 1-4,6-9<br>1-9 |
| Y | JP 2007-181406 A  (Ito En, Ltd.),<br>19 July, 2007 (19.07.07),<br>Page 10; table 6<br>& WO 2007/77746 A | 1-9 |
| Y | Iwai, Kazuya et al, In vitro antioxidative effects and tyrosinase inhibitory activities of seven hydroxycinnamoyl derivatives in green coffee beans, Journal of Agricultural and Food Chemistry, Vol. 52, No.15, pp.4893-4898, abstract, Fig.4 | 1-9 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>08 December, 2008 (08.12.08) | Date of mailing of the international search report<br>22 December, 2008 (22.12.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2008/067528 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 9-143465 A  (Dainippon Ink And Chemicals, Inc.), 03 June, 1997 (03.06.97), (Family: none) | 1-9 |
| Y | JP 63-502434 A  (Ergo Forschungsgesellschaft mbH), 14 September, 1988 (14.09.88), & US 4872987 A          & EP 258297 A & WO 87/4704 A1          & DE 3762338 A & BR 8705410 A | 1-9 |

Form PCT/ISA/210 (continuation of second sheet) (April 2007)

<table>
<tr><td>**INTERNATIONAL SEARCH REPORT**</td><td>International application No.<br>PCT/JP2008/067528</td></tr>
</table>

Continuation of A. CLASSIFICATION OF SUBJECT MATTER
(International Patent Classification (IPC))

C07C67/56(2006.01)i, C07C69/732(2006.01)i, C09K15/08(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006102477 A
- JP 2003306417 A
- JP 2006116433 A
- JP 53018772 A
- JP 4145049 A
- JP 51091368 A
- JP 2006306799 A
- JP 10212221 A
- JP 2007249759 A

**Non-patent literature cited in the description**

- Investigation on the Actual Using State of Food Flagrance Compounds in Japan. Japan Flavor and Fragrance Material Association, 2000
- Synthesized Flavoring, Chemistry and Merchandise Information. The Chemical Daily Co., Ltd, 06 March 1996
- **Steffen Arctender.** *Perfume and Flavor Chemicals,* 1969, vol. 1, 2
- *Analyst,* 1984, vol. 109, 263-266